# EUROPÄISCHE PATENTANMELDUNG

(11) **EP 2 103 598 A1**
(43) Veröffentlichungstag der Anmeldung: **23.09.2009**
(21) Anmeldenummer: 09005497.4
(22) Anmeldetag: 02.06.2003
(51) Int. Cl.: C07C 317/46, C07C 317/48, C07C 317/44, A61K 31/155, A61P 9/10, C07C 35/48, C07C 211/37

(54) **Fluorierte Cycloalkyl-derivatisierte Benzoylguanidine, Verfahren zu ihre Herstellung, ihre Verwendung als Medikament, sowie sie enthaltendes Medikament**

(30) Priorität: 13.06.2002 DE 10226462
(62) Teilanmeldung aus: 03759904.0
(71) Anmelder: Sanofi-Aventis Deutschland GmbH, 65929 Frankfurt am Main (DE)
(72) Erfinder: Kleemann, Heinz-Werner, Dr., 65926 Frankfurt am Main (DE)

(57) **Zusammenfassung**

Fluorierte Cycloalkyl-derivatisierte Benzoylguanidine der Formel I 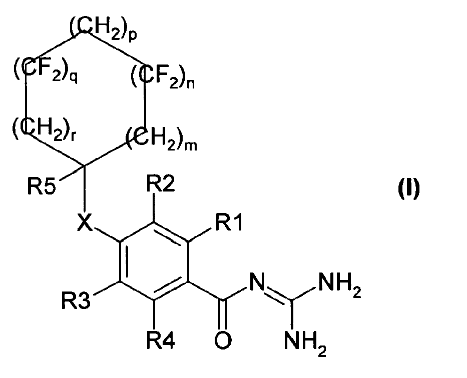 sind als antiarrhythmische Arzneimittel mit cardioprotektiver Komponente zur Infarktprophylaxe und der Infarktbehandlung sowie zur Behandlung der angina pectoris geeignet.

Sie inhibieren auch präventiv die pathophysiologischen Vorgänge beim Entstehen ischämisch induzierter Schäden, insbesondere bei der Auslösung ischämisch induzierter Herzarrhythmien und der Herzinsuffizienz.

## Beschreibung

Die Erfindung betrifft Verbindungen der Formel I 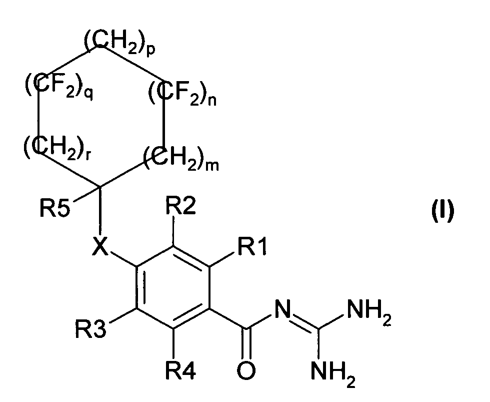 worin bedeuten:
- X: Sauerstoff, Schwefel oder NR6;
R6 Wasserstoff, Alkyl mit 1, 2, 3 oder 4 C-Atomen oder (CH₂)ₖ-CF₃;
k k 0, 1, 2 oder 3;
- m: Null, 1, 2 oder 3;
- n: Null, 1, 2 oder 3;
- p: Null, 1, 2 oder 3;
- q: 1, 2 oder 3;
- r: Null, 1, 2 oder 3;
- wobei: die Summe von m, n, p, q und r mindestens 2 ist;
- R1: Wasserstoff, Alkyl mit 1, 2, 3 oder 4 C-Atomen, F, CI, -OR(7), -NR(8)R(9) oder -CₛF₂ₛ₊₁ ;
R(7), R(8) und R(9) unabhängig voneinander Wasserstoff, Alkyl mit 1, 2 oder 3 C-Atomen oder (CH₂)ₜ-CF₃;
s 1, 2, 3 oder 4;
t 0, 1, 2, 3 oder 4;
- R2: Wasserstoff, F, CI, Alkyl mit 1, 2, 3 oder 4 C-Atomen oder CF₃;
- R3: Wasserstoff, F, CI, Alkyl mit 1, 2, 3 oder 4 C-Atomen, CF₃ oder SOᵤR10;
- u: Null, 1 oder 2;
- R10: Alkyl mit 1, 2, 3 oder 4 C-Atomen oder NR11R12;
- R11 und R12: unabhängig voneinander Wasserstoff oder Alkyl mit 1, 2, 3 oder 4 C-Atomen;
- R4: Wasserstoff, Alkyl mit 1, 2, 3 oder 4 C-Atomen, F, CI, -OR(13), -NR(14)R(15) oder -CᵥF₂ᵥ₊₁;
- R(13), R(14) und R(15): unabhängig voneinander Wasserstoff, Alkyl mit 1, 2 oder 3 C-Atomen oder (CH₂)_{w}-CF₃;
- v: 1, 2, 3 oder 4;
- w: 0, 1, 2, 3 oder 4;
- R5: Wasserstoff oder F;
sowie deren pharmakologisch verträgliche Salze.

Bevorzugt sind Verbindungen der Formel I, worin bedeuten:
- X: Sauerstoff, Schwefel oder NR6;
- R6: Wasserstoff, Methyl oder CH₂-CF₃;
- m: Null, 1 oder 2;
- n: Null, 1 oder 2;
- p: Null, 1 oder 2;
- q: 1 oder 2;
- r: Null, 1 oder 2;
wobei die Summe von m, n, p, q und r mindestens 2 ist;
- R1: Wasserstoff, Methyl, F, CI, -OR(7), -NR(8)R(9) oder -CF₃;
- R(7), R(8) und R(9): unabhängig voneinander Wasserstoff, Methyl, CF₃ oder CH₂-CF₃;
- R2: Wasserstoff, F, CI, Methyl oder CF₃;
- R3: Wasserstoff, F, CI, Alkyl mit 1, 2, 3 oder 4 C-Atomen, CF₃, SO₂CH₃ oder SO₂NH₂;
- R4: Wasserstoff, Methyl, F, CI, -OR(13), -NR(14)R(15) oder -CF₃;
- R(13), R(14) und R(15): unabhängig voneinander Wasserstoff, Methyl, CF₃ oder CH₂-CF₃;
- R5: Wasserstoff oder F;
sowie deren pharmakologisch verträgliche Salze.

Besonders bevorzugt sind Verbindungen der Formel I, worin bedeuten:
- X: Sauerstoff, Schwefel oder NR6;
- R6: Wasserstoff, Methyl oder CH₂-CF₃;
- m: Null oder 1;
- n: Null, 1 oder 2;
- p: Null oder 1;
- q: 1 oder 2;
- r: Null oder 1;
- wobei: die Summe von m, n, p, q und r mindestens 2 ist;
- R1: Wasserstoff, Methyl, F, CI, -OR(7), -NR(8)R(9) oder -CF₃;
- R(7): Methyl, CF₃ oder CH₂-CF₃;
- R(8) und R(9): unabhängig voneinander Wasserstoff, Methyl oder CH₂-CF₃;
- R2: Wasserstoff, F oder CI;
- R3: CF₃, SO₂CH₃ oder SO₂NH₂;
- R4: Wasserstoff;
- R5: Wasserstoff oder F;
sowie deren pharmakologisch verträgliche Salze.

Ganz besonders bevorzugt sind folgende Verbindungen der Formel I, ausgewählt aus der Gruppe:
N-[4-(3,3-Difluoro-cyclobutoxy)-5-methansulfonyl-2-methyl-benzoyl]-guanidin,
N-[4-(3,3-Difluoro-cyclobutylamino)-5-methansulfonyl-2-methyl-benzoyl]-guanidin,
N-{4-[(3,3-Difluoro-cyclobutyl)-methyl-amino]-5-methansulfonyl-2-methyl-benzoyl}-guanidin
   und
N-{4-[(3,3-Difluoro-cyclobutyl)-methyl-amino]-5-ethansulfonyl-2-methyl-benzoyl}-guanidin
sowie deren pharmazeutisch verträgliche Salze.

Die Verbindungen der Formel I, zum Beispiel die Substituenten R1 bis R4, enthalten ein oder mehrere Asymmetriezentren, so können diese unabhängig voneinander sowohl S als auch R konfiguriert sein. Die Verbindungen können als optische Isomere, als Diastereomere, als Racemate oder als Gemische derselben vorliegen.
Die vorliegende Erfindung umfasst alle tautomeren Formen der Verbindungen der Formel I und II.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl) und tert-Butyl (= 1,1-Dimethylethyl). Bevorzugte Alkylreste sind Methyl, Ethyl, n-Propyl und Isopropyl. In Alkylresten können ein oder mehrere, zum Beispiel 1, 2, 3, 4 oder 5, Wasserstoffatome durch Fluoratome substituiert sein. Beispiele für solche Fluoralkylreste sind Trifluormethyl, 2,2,2-Trifluorethyl und Pentafluorethyl. Substituierte Alkylreste können in beliebigen Positionen substituiert sein.

Bevorzugt sind Verbindungen der Formel I, in denen die Summe aus m, n, o, p, q und r 2 bis 6 beträgt.

Die Erfindung betrifft außerdem ein Verfahren zur Herstellung einer Verbindung der Formel I, das dadurch gekennzeichnet ist, dass man eine Verbindung der Formel II 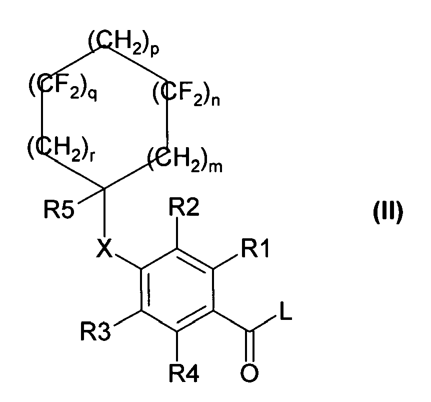 worin R(1) bis R(5) sowie m bis r die angegebene Bedeutung besitzen und L für eine leicht nucleophil substituierbare Fluchtgruppe steht, mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in dem Fachmann bekannter Weise aus den zugrundeliegenden Carbonsäurechloriden (Formel II, L = CI), die man ihrerseits wiederum in dem Fachmann bekannter Weise aus den zugrundeliegenden Carbonsäuren (Formel II, L = OH) beispielsweise mit Thionylchlorid herstellen kann.

Neben den Carbonsäurechloriden der Formel II (L = CI) lassen sich auch weitere aktivierte Säurederivate der Formel II in an sich bekannter Weise direkt aus den zugrundeliegenden Benzoesäuren (Formel II, L = OH) herstellen, wie die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride mit Cl-COOC₂H₅ oder Tosylchlorid in Gegenwart von Triethylamin in einem inerten Lösungsmittel, wie auch Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluorborat ("TOTU") [Proceedings of the 21. European Peptide Symposium, Peptides 1990, Editors E. Giralt and D. Andreu, Escom, Leiden, 1991] möglich sind. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in dem Fachmann bekannter Weise in einem protischen oder aprotischen polaren aber inerten organischen Lösungsmittel. Dabei haben sich bei der Umsetzung der Benzoesäuremethylester (II, L = OCH₃) mit Guanidin Methanol, Isopropanol oder THF bei Temperaturen von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmittel wie THF, Dimethoxyethan, Dioxan oder DMF gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, zum Beispiel in Form von überschüssigem Guanidin zur Abbindung der Halogenwasserstoffsäure.

Die Carbonsäurederivate der Formel II können aus Verbindungen der Fomel III hergestellt werden. Ein Teil der zugrundeliegenden Benzoesäurederivate der Formel III ist bekannt und in der Literatur beschrieben. Die unbekannten Verbindungen der Formel III können nach literaturbekannten Methoden hergestellt werden.

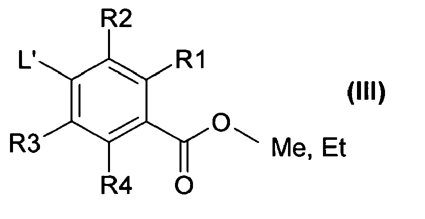

Die Einführung der Fluorcycloalkyl-Nucleophile in die 4-Position erfolgt durch nucleophile aromatische Substitution. Hierbei werden geeignet geschützte Benzoesäure-Derivate der Formel III, wie zum Beispiel die Methyl- oder Ethylester eingesetzt. L' bezeichnet eine durch nucleophile aromatische Substitution leicht substituierbare Fluchtgruppe wie F, CI, Br, I, oder O-SO₂CF₃.

Die erhaltenen Benzoesäurederivate der Formel II werden anschließend nach einer der oben beschriebenen Verfahrensvarianten zu erfindungsgemäßen Verbindungen der Formel I umgesetzt.

Benzoylguanidine der Formel I sind im allgemeinen schwache Basen und können Säure unter Bildung von Salzen binden. Als Säureadditionssalze kommen Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.
Die Verbindungen der Formel 1 sind substituierte Acylguanidine und inhibieren den zellulären Natrium-Protonen-Antiporter (Na⁺/H⁺-Exchanger, NHE).

Den erfindungsgemäßen Verbindungen der Formel I zeichnen sich gegenüber bekannten Benzoylguanidinen durch ungewöhnlich günstige ADME(Absorption-Distribution-Metabolism-Excretion)-Eigenschaften, verbunden mit ausgezeichneter Wirksamkeit in der Inhibition des Na⁺/H⁺-Austauschs aus; bedingt werden diese vorteilhaften Eigenschaften durch die fluorierte Cycloalkylgruppe.

Im Gegensatz zu bekannten Acylguanidinen zeigen die hier beschriebenen Verbindungen keine unerwünschten und nachteiligen salidiuretischen Eigenschaften.

Aufgrund der NHE-inhibitorischen Eigenschaften eignen sich die Verbindungen der Formel I und/oder deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHEbedingten Schädigungen verursacht werden.

Da NHE Inhibitoren in überwiegender Weise über ihre Beeinflussung der zellulären pH-Regulation wirken, können diese generell in günstiger Weise mit anderen, den intrazellulären pH-Wert regulierenden Verbindungen kombiniert werden, wobei Inhibitoren der Enzymgruppe der Carboanhydrasen, Inhibitoren der Bicarbonationen transportierenden Systeme wie des Natrium-Bicarbonat-Cotransporters (NBC) oder des Natrium-abhängigen Chlorid-Bicarbonat-Austauschers (NCBE), sowie NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.
So eignen sich die erfindungsgemäßen Inhibitoren des NHE zur Behandlung von Krankheiten, die durch Ischämie und durch Reperfusion hervorgerufen werden.

Die hier beschriebenen Verbindungen sind infolge ihrer pharmakologischen Eigenschaften als antiarrhythmische Arzneimittel geeignet.
Durch ihre cardioprotektive Komponente eignen sich die NHE-Inhibitoren der Formel I und/oder deren pharmazeutisch verträgliche Salze hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I und/oder deren pharmazeutisch verträgliche Salze infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die erfindungsgemäßen Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Es zeigte sich, dass die erfindungsgemäßen Verbindungen außerordentlich wirksame Arzneimittel sind gegen lebensbedrohliche Arrhythmien. Kammerflimmern wird beendet und der physiologische Sinus-Rhythmus des Herzens wiederhergestellt.

Da NHE1-Inhibitoren menschliches Gewebe und Organe, insbesondere das Herz, nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist die kombinierte Verabreichung mit Verbindungen der Formel I und/oder deren pharmazeutisch verträgliche Salze geeignet, die cytotoxischen, insbesondere cardiotoxischen Nebenwirkungen der genannten Verbindungen zu inhibieren. Durch die Verminderung der cytotoxischen Effekte, insbesondere der Cardiotoxizität, infolge Ko-Medikation mit NHE1-Inhibitoren kann außerdem die Dosis der cytotoxischen Therapeutika erhöht und/oder die Medikation mit solchen Arzneimitteln verlängert werden. Der therapeutische Nutzen einer solchen cytotoxischen Therapie kann durch die Kombination mit NHE-Inhibitoren erheblich gesteigert werden.

Außerdem können die erfindungsgemäßen NHE1-Inhibitoren der Formel I und/oder deren pharmazeutisch verträgliche Salze bei einer herzschädigenden Überproduktion von Schilddrüsenhormonen, der Thyreotoxikose, oder bei der externen Zufuhr von Schilddrüsenhormonen Verwendung finden. Die Verbindungen der Formel I und/oder deren pharmazeutisch verträgliche Salze eignen sich somit zur Verbesserung der Therapie mit cardiotoxischen Arzneimitteln.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie zum Beispiel zur Behandlung des Schlaganfalls oder des Hirnödems geeignet sind.

Die Verbindungen der Formel I und/oder deren pharmazeutisch verträgliche Salze eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychische Depressionszustände, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäß verwendeten Verbindungen der Formel I und/oder deren pharmazeutisch verträgliche Salze ebenfalls zur Behandlung von Formen des Schocks, wie beispielweise des allergischen, cardiogenen, hypovolämischen und des bakteriellen Schocks.

Die Verbindungen der Formel I und/oder deren pharmazeutisch verträgliche Salze können ebenfalls zur Prävention und zur Behandlung thrombotischer Erkrankungen verwendet werden, da sie als NHE-Inhibitoren die Plättchenaggregation selbst inhibieren können. Darüberhinaus können sie die nach Ischämie und Reperfusion stattfindende überschießende Freisetzung von Entzündungs- und Gerinnungsmediatoren, insbesondere des *von Willebrand Faktors* und thrombogener Selectin-Proteine hemmen bzw. verhindern. Damit kann die pathogene Wirkung bedeutender thrombogener Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan Rezeptor-Antagonisten, Phosphodiesterase-Inhibitoren, Factor-VIIa-Antagonisten, Clopidogrel, Ticolopidin usw. Eine kombinierte Anwendung der vorliegenden NHE-Inhibitoren mit NCBE-Inhibitoren und/oder mit Inhibitoren der Carboanhydrase, wie beispielsweise mit Acetazolamid, ist besonders günstig.

Darüber hinaus zeichnen sich die erfindungsgemäß verwendeten Verbindungen der Formel I und/oder deren pharmazeutisch verträgliche Salze durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I und/oder deren pharmazeutisch verträgliche Salze als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden.

Es konnte gezeigt werden, dass durch die erfindungsgemäßen Verbindungen die Zellmigration inhibiert wird. Daher kommen die Verbindungen der Formel I und/oder deren pharmazeutisch verträgliche Salze als wertvolle Therapeutika für Krankheiten in Frage, bei denen die Zellmigration eine primäre oder sekundäre Ursache darstellt, wie beispielsweise Krebserkrankungen mit ausgeprägter Neigung zur Metastasierung.

Die Verbindungen der Formel I und/oder deren pharmazeutisch verträgliche Salze zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.
Sie können somit zur Behandlung von Organhypertrophien und -hyperplasien, beispielsweise des Herzens und der Prostata verwendet werden. Sie sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

Da der NHE bei essentiellen Hypertonikern signifikant erhöht ist, eignen sich die Verbindungen der Formel I und/oder deren pharmazeutisch verträgliche Salze zur Prävention und zur Behandlung des Bluthochdrucks und von Herz-Kreislauferkrankungen.
Dabei können sie allein oder mit einem geeigneten Kombinations- und Formulierungspartner zur Bluthochdruckbehandlung und von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amiloride, Triamteren, Spironolacton oder Epleron, kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin- Rezeptoragonisten, Inhibitoren und Aktivatoren von Kaliumkanälen wie Glibenclamide, Glimepirid, Diazoxide, Cromokalim, Minoxidil und deren Derivate, Aktivatoren des mitochondrialen ATP-sensitiven Kaliumkanals (mitoK(ATP) Kanal), Inhibitoren des Kv1.5 usw.

Es zeigte sich, dass NHE1 Inhibitoren der Formel I und/oder deren pharmazeutisch verträgliche Salze eine signifikante antiphlogistische Wirkung haben und somit als Antiinflammatorika verwendet werden können. Dabei fällt die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet. Auch können die erfindungsgemäßen Verbindungen verwendet werden bei der Behandlungen von Erkrankungen, die durch Protozoen verursacht werden, von Malaria und der Hühnercoccidiose.

Es wurde außerdem gefunden, dass Verbindungen der Formel I und/oder deren pharmazeutisch verträgliche Salze eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, dass NHE1 Inhibitoren bezüglich der Beeinflussung der Serumlipidspiegel wertvolle therapeutisch verwertbare Eigenschaften zeigen. So erniedrigen sie signifikant die erhöhte Serum Konzentrationen von LDL und VLDL, wie sie beispielweise durch erhöhte diätetische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie zum Beispiel beim Diabetes vorkommen. Darüber hinaus führen die Verbindungen der Formel I und/oder deren pharmazeutisch verträgliche Salze zu einer deutlichen Reduktion der durch Stoffwechselanomalien induzierten Infarkte und insbesondere zu einer signifikanten Verminderung der induzierten Infarktgröße und dessen Schweregrades.

Die erfindungsgemäßen Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I und/oder deren pharmazeutisch verträgliche Salze mit einem blutfettspiegelsenkenden Wirkstoff, bevorzugt mit einem HMG-CoA-Reduktaseinhibitor (zum Beispiel Lovastatin oder Pravastatin), wobei letzterer eine hypolipidämische Wirkung herbeiführt und dadurch die hypolipidämischen Eigenschaften des NHE-Inhibitors der Formel I und/oder deren pharmazeutisch verträgliche Salze steigert, erweist sich als günstige Kombination mit verstärkter Wirkung und vermindertem Wirkstoffeinsatz.

So führen Verbindungen der Formel I und/oder deren pharmazeutisch verträgliche Salze zu einen wirksamen Schutz gegen Endothelschädigungen unterschiedlicher Genese. Mit diesem Schutz der Gefäße gegen das Syndrom der endothelialen Dysfunktion sind Verbindungen der Formel I und/oder deren pharmazeutisch verträgliche Salze wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem wurde gefunden, dass Benzoylguanidine der Formel I und/oder deren pharmazeutisch verträgliche Salze geeignet in der Behandlung des nichtinsulinabhängigen Diabetes (NIDDM) sind, wobei die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburide, Glimepiride, Tolbutamid usw., einem Glucosidase Inhibitor, einem PPAR Agonisten, wie Rosiglitazone, Pioglitazone etc., mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken die Verbindungen der Formel I und/oder deren pharmazeutisch verträgliche Salze der Entstehung diabetischer Spätkomplikationen entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den soeben unter NIDDM-Behandlung beschriebenen antidiabetischen Arzneimitteln vorteilhaft kombiniert werden. Der Kombination mit einer günstigen Darreichungsform von Insulin dürfte dabei eine besondere Bedeutung zukommen.

Die erfindungsgemäßen NHE-Inhibitoren der Formel I und/oder deren pharmazeutisch verträgliche Salze zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die unabhängig von akuten Mangeldurchblutungszuständen sind und bei normalen, nichtischämischen Bedingungen auftreten. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. Die Verbindungen der Formel I und/oder deren pharmazeutisch verträgliche Salze eignen sich somit hervorragend zur Behandlung und Prävention der altersbedingt fortschreitenden endothelialen Dysfunktion, insbesondere von claudicatio intermittens.

Beispiel einer weiteren den Altersprozess charakterisierenden Messgröße ist die Abnahme der Kontraktilität des Herzens und die Abnahme der Anpassung des Herzens an eine geforderte Pumpleistung des Herzens. Diese verminderte Herzleistungsfähigkeit als Folge des Alterungsprozesses ist in den meisten Fällen verbunden mit einer Dysfunktion des Herzens, die unter anderem durch eine Einlagerung von Bindegewebe ins Herzgewebe verursacht wird. Diese Bindegewebseinlagerung ist gekennzeichnet durch eine Zunahme des Herzgewichtes, durch eine Vergrößerung des Herzens und durch eine eingeschränkte Herzfunktion. Es war überraschend, dass eine derartige Alterung des Organs Herz nahezu komplett inhibiert werden konnte. Die Verbindungen der Formel I und/oder deren pharmazeutisch verträgliche Salze eignen sich somit hervorragend zur Behandlung und Prävention der Herzinsuffizienz, des congestive heart failure (CHF).

Während in den vorangegangenen Patenten und Patentanmeldungen die Behandlung von unterschiedlichen, bereits eingetretenen Formen von Krebserkrankungen beansprucht wurden, war es nun außerordentlich überraschend, dass nicht nur die bereits eingetretene Krebserkrankung durch Proliferationshemmung geheilt werden kann, sondern dass auch die altersbedingte Entstehungshäufigkeit von Krebs durch NHE-Inhibitoren verhindert und hochsignifikant verzögert wird. Besonders bemerkenswert ist der Befund, dass altersbedingt auftretenden Erkrankungen aller Organe und nicht nur bestimmter Krebsformen unterbunden bzw. hochsignifikant verzögert auftreten. Die Verbindungen der Formel I und/oder deren pharmazeutisch verträgliche Salze eignen sich somit hervorragend zur Behandlung und insbesondere der Prävention von altersbedingten Formen von Krebs.

Es wird nun nicht nur eine über das statistische Normalmaß hinaus, zeitlich hochsignifikant verschobene Verzögerung des Eintretens altersbedingter Erkrankungen aller untersuchten Organe einschließlich Herz, Gefäße, Leber usw., sowie eine hochsignifikante Verzögerung von Alterskrebs festgestellt. Vielmehr kommt es auch überraschenderweise zu einer Lebensverlängerung in einem Ausmaß, das bislang durch keine andere Medikamentengruppe bzw. durch irgendwelche Naturstoffe erreicht werden konnte. Diese einzigartige Wirkung der NHE-Inhibitoren ermöglicht es auch, neben der alleinigen Wirkstoffanwendung an Mensch und Tier diese NHE-Inhibitoren mit anderen gerontologisch verwendeten Wirkprinzipien, Maßnahmen, Substanzen und Naturstoffen zu kombinieren, denen ein anderer Wirkmechanismus zugrunde liegt. Derartige in der gerontologischen Therapie verwendete Wirkstoffklassen sind: insbesondere Vitamine und antioxidativ wirksame Stoffe. Da eine Korrelation zwischen kalorischer Belastung bzw. Nahrungsaufnahme und Alterungsprozeß besteht, kann die Kombination mit diätetischen Maßnahmen zum Beispiel mit Appetitzüglern erfolgen. Ebenso kann eine Kombination mit blutdrucksenkenden Medikamenten, wie mit ACE-Hemmern, Angiotensinrezeptorantagonisten, Diuretika, Ca⁺²-Antagonisten etc. oder mit stoffwechselnormalisierenden Medikamenten, wie Cholesterinsenkern, gedacht werden.
Die Verbindungen der Formel I und/oder deren pharmazeutisch verträgliche Salze eignen sich somit hervorragend zur Prävention altersbedingter Gewebsveränderungen und zur Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Beansprucht wird weiterhin ein Heilmittel für die humane, veterinäre oder phytoprotektive Anwendung enthaltend eine wirksame Menge einer oder mehrerer Verbindungen der Formel I und/oder deren pharmazeutisch verträgliche Salze, zusammen mit pharmazeutisch annehmbaren Träger- und Zusatzstoffen, allein oder in Kombination mit anderen pharmakologischen Wirkstoffen oder Arzneimitteln.

Arzneimittel, die eine Verbindung I enthalten, können dabei beispielsweise oral, parenteral, intravenös, rektal, perkutan oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässerige, alkoholische oder ölige Lösungen. Als inerte Träger können zum Beispiel Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen in Lösung, Suspension oder Emulsion gebracht. Als Lösungsmittel kommen zum Beispiel in Frage: Wasser, physiologische Kochsalzlösung oder Alkohole, zum Beispiel Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet zum Beispiel Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere Dosierungen notwendig sein. Insbesondere bei i.v. Anwendung, etwa bei einem Infarktpatienten auf der Intensivstation können bis zu 700 mg pro Tag notwendig werden. Die tägliche Dosis kann auf mehrere, zum Beispiel bis zu 4 Einzeldosen, verteilt werden.

Liste der Abkürzungen:
- ADME: Absorption-Distribution-Metabolism-Excretion
- CDI: Di-imidazol-1-yl-methanon
- DIP: Diisopropylether
- DMF: N,N-Dimethylformamid
- EE: Ethylacetat (EtOAc)
- EI: electron impact
- eq.: Äquivalent
- ES: Elektrospray-lonisierung
- Et: Ethyl
- HEP: n-Heptan
- KOtBu: Kalium-2-methyl-propan-2-olat
- Me: Methyl
- MeOH: Methanol
- mp: Schmelzpunkt
- NMP: 1-Methyl-pyrrolidin-2-on
- MTB: 2-Methoxy-2-methyl-propan
- RT: Raumtemperatur
- THF: Tetrahydrofuran

### Beispiel 1: N-[4-(3,3-Difluor-cyclobutoxy)-5-methansulfonyl-2-methyl-benzoyl]-guanidin

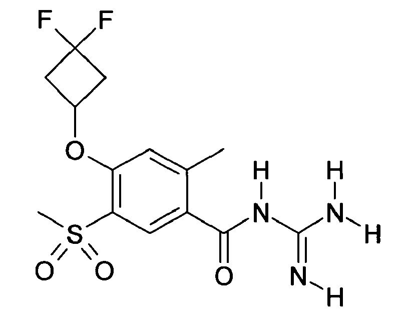

### a) (3,3-Difluor-cyclobutoxymethyl)-benzol

20.0 g 3-Benzyloxy-cyclobutanon (Bull. Chem. Soc. Jpn. (1984), 57(6), 1637) wurden in 150 ml CH₂Cl₂ gelöst und eine Lösung von 25.0 g [Bis(2-methoxyethyl)amino]-schwefeltrifluorid in 30 ml CH₂Cl₂ bei RT zugetropft. 5 h wurde bei RT gerührt und anschließend 12.0 g Diethylamino-schwefeltrifluorid zugegeben. Weitere 20 h wurde bei RT gerührt, anschließen das Reaktionsgemisch 3 mal mit je 100 ml Wasser gewaschen. Über Na₂SO₄ wurde getrocknet und das Solvens im Vakuum entfernt. Chromatographie an Kieselgel mit EE/HEP 3:1 und anschließende Kugelrohrdestillation lieferte 27.6 g eines farblosen Öls.

### b) 3,3-Difluor-cyclobutanol

22.4 g (3,3-Difluor-cyclobutoxymethyl)-benzol werden in 100 ml Diethylether gelöst und 1.4 g 10 % Pd/C zugegeben. 5 h lang wurde bei RT unter 20 bar H₂ hydriert. Der Katalysator wurde mit 10 ml Diethylether gewaschen und die Lösung destilliert. Man erhielt 14.0 g des Produktes (Sdp. 80°C), als Mischung mit Diethylether und Toluol. Diese Mischung wurde ohne weitere Reinigung umgesetzt.

### c) 4-(3,3-Difluor-cyclobutoxy)-5-methansulfonyl-2-methyl-benzoesäure-methylester

370 mg 4-Fluor-5-methansulfonyl-2-methyl- benzoesäure-methylester, 297 mg 3,3-Difluor-cyclobutanol und 1.47 g Cs₂CO₃ wurden in 10 ml wasserfreiem NMP gelöst und 4 h bei 60°C gerührt. Anschließend wurde das Reaktionsgemisch mit 125 ml einer halbkonzentrierten wäßrigen NaHCO₃-Lösung verdünnt und 3 mal mit je 100 ml EE extrahiert. Über Na₂SO₄ wurde getrocknet und das Solvens im Vakuum entfernt. An Kieselgel wurde mit DIP chromatographiert und man erhielt 380 mg eines farblosen Öls.
Rf (DIP) = 0.21 MS (DCI) : 335

### d) N-[4-(3,3-Difluor-cyclobutooy)-5-methansulfonyl-2-methyl-benzoyl]-guanidin

566 mg Guanidinium-chlorid wurden in 5 ml wasserfreiem DMF gelöst und zu einer Lösung von 604 mg KOtBu in 5 ml wasserfreiem DMF zugegeben. Diese so hergestellte Lösung von Guanidin in DMF wurde zu einer Lösung von 360 mg 4-(3,3-Difluor-cydobutoxy)-5-methansulfonyl-2-methyl-benzoesäure-methylester in 5 ml DMF gegeben und 24 h bei RT gerührt. Das Reaktionsgemisch wurde mit 125 ml einer halbkonzentrierten wäßrigen NaHCO₃-Lösung verdünnt und 3 mal mit je 80 ml EE extrahiert. Über Na₂SO₄ wurde getrocknet und das Solvens im Vakuum entfernt. Chromatographie an Kieselgel mit EE/MeOH 5:1 lieferte 175 mg farbloser Kristalle, mp 273°C (unter Zersetzung).
Rf (EE/MeOH 5:1) = 0.50 MS (ES⁺) : 362

### Beispiel 2

### N-[4-(3,3-Difluoro-cyclobutylamino)-5-methansulfonyl-2-methyl-benzoyl]-guanidin

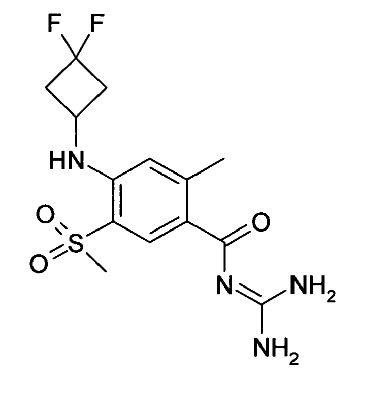

### a) 3,3-Difluoro-cydobutylamin, Hydrochlorid

16.7 g 3,3-Difluoro-cyclobutancarbonsäure (J.Org.Chem. 1987,52,1872) wurden in 180 ml CHCl₃ gelöst und 36 ml H₂SO₄ (98%) zugegeben.Das Gemisch wurde auf 50°C erwärmt und bei dieser Temperatur portionsweise während 45 Minuten 16 g NaN₃ zugesetzt. 2 h wurde bei 50°C nachgerührt, dann wurde das Gemisch auf Raumtemperatur abgekühlt und schließlich auf 250 g Eis gegossen. 3 mal wurde mit je 100 ml Diethylether extrahiert und so 0.7 g unumgesetzte 3,3-Difluoro-cyclobutancarbonsäure zurückisoliert. Die wäßrige Phase wurde mit wäßriger NaOH-Lösung auf pH = 12-13 eingestellt und 3 mal mit je 100 ml CH₂Cl₂ extrahiert. Die organische Phase wurde noch mit 100 ml Wasser gewaschen, anschließend mit 130 ml einer 2N wäßrigen HCl-Lösung versetzt und die flüchtigen Bestandteile im Vakuum entfernt. Man erhielt 15.3 g eines farblosen Feststoffs, mp 315°C (Zersetzung).

### b) N-(3,3-Difluoro-cyclobutyl)-methansulfonamid

0.60 g 3,3-Difluoro-cyclobutylamin, Hydrochlorid wurden in 40 ml CH₂Cl₂ suspendiert und bei RT 2.9 ml Triethylamin zugegeben. Dabei erhielt man eine klare Lösung. Anschließend wurde langsam bei RT 1.0 ml Methansulfonylchlorid zugetropft und das Reaktionsgemisch 16 h bei dieser Temperatur stehen gelassen. Dann wurden die flüchtigen Bestandteile im Vakuum entfernt, der Rückstand mit 200 ml EE sowie 100 ml einer gesättigten wäßrigen Na₂CO₃-Lösung aufgenommen und verteilt. Die organische Phase wurde anschließend noch 2 mal mit je 20 ml einer gesättigten wäßrigen NaHSO₄-Lösung und 2 mal mit je 30 ml einer gesättigten wäßrigen Na₂CO₃-Lösung gewaschen. Über MgSO₄ wurde getrocknet und das Solvens im Vakuum entfernt. Man erhielt 700 mg eines farblosen Harzes.
Rf (EE) = 0.28 MS (DCI) : 186

### c) 4-(3,3-Difluoro-cyclobutylamino)-5-methansulfonyl-2-methyl-benzoesäure-methylester

0.70 g N-(3,3-Difluoro-cyclobutyl)-methansulfonamid, 0.93 g 4-Fluoro-5-methansulfonyl-2-methyl-benzoesäure-methylester und 1.5 ml N"-tert-Butyl-N,N,N',N'-tetramethyl-guanidin wurden in 10 ml NMP (wasserfrei) gelöst und 6 h bei 150°C gerührt. Das Reaktionsgemisch wurde auf RT abgekühlt, mit 200 ml EE verdünnt und zunächst 3 mal mit je 20 ml einer gesättigten wäßrigen NaHSO₄-Lösung, dann 3 mal mit je 30 ml einer gesättigten wäßrigen Na₂CO₃-Lösung gewaschen. Über MgSO₄ wurde getrocknet und das Solvens im Vakuum entfernt. Chromatographie an Kieselgel mit DIP lieferte 220 mg eines farblosen Schaumes.
Rf (DIP) = 0.31 MS (ES⁺) : 334

### d) 4-(3,3-Difluoro-cyclobutylamino)-5-methansulfonyl-2-methyl-benzoesäure

210 mg 4-(3,3-Difluoro-cyclobutylamino)-5-methansulfonyl-2-methyl-benzoesäure-methylester wurden in 10 ml Dioxan gelöst und mit 0.47 ml einer 2N wäßrigen NaOH-Lösung versetzt. 18 h wurde bei RT gerührt, anschließend die Solventien im Vakuum entfernt. Der Rückstand wurde mit 10 ml Wasser aufgenommen, mit wäßriger HCl-Lösung auf pH = 2 gestellt und 3 mal mit je 20 ml EE extrahiert. Über MgSO₄ wurde getrocknet und das Solvens im Vakuum entfernt. Man erhielt 196 mg eines amorphen Feststoffs.
Rf (EE) = 0.40 MS (ES⁻) : 318

### e) N-[4-(3,3-Difluoro-cyclobutylamino)-5-methansulfonyl-2-methyl-benzoyl]-guanidin

40 mg 4-(3,3-Difluoro-cyclobutylamino)-5-methansulfonyl-2-methyl-benzoesäure wurden in 1 ml DMF (wasserfrei) gelöst, bei RT mit 26 mg CDI versetzt und bei RT 6 h lang gerührt und man erhielt das intermediäre Imidazolid. Daneben wurden 72 mg Guanidin-Hydrochlorid zusammen mit 70 mg KO*t*Bu 30 Minuten lang bei RT in 1 ml DMF (wasserfrei) gerührt. Diese Lösung der Guanidin-Base wurde anschließend zur Lösung des Imidazolids gegeben und 18h bei RT stehen gelassen. Man verdünnte anschließend mit 50 ml Wasser und stellte mit verdünnter wäßriger HCl-Lölsung den pH auf pH=8. 3 mal wurde mit je 10 ml EE extrahiert, über MgS04 wurde getrocknet und das Solvens im Vakuum entfernt. Man erhielt 37 mg eines amorphen Feststoffs.
Rf (EE/MeOH 10:1) = 0.12 MS (ES⁺) : 360

Die NHE-1 Hemmung wurde wie folgt bestimmt:
FLIPR-Assay zur Bestimmung von NHE-1 Inhibitoren mittels Messung der pHᵢ-Erholung in transfizierten Zelllinien, die den humanen NHE-1 exprimieren

Der Assay wird im FLIPR (Fluorescent imaging plate reader) mit schwarzwandigen 96-Well-Mikrotiterplatten mit klarem Boden durchgeführt. Die transfizierten Zelllinien, welche die verschiedenen NHE-Subtypen exprimieren (die parentale Zelllinie LAP-1 weist als Folge von Mutagenese und anschließender Selektion keine endogene NHE-Aktivität auf), werden am Vortag mit einer Dichte von ∼25.000 Zellen / Well ausgesät. [Das Wachstumsmedium der transfizierten Zellen (Iscove +10 % fötales Kälberserum) enthält zusätzlich G418 als Selektionsantibiotikum, um die Anwesenheit der transfizierten Sequenzen sicherzustellen.]

Der eigentliche Assay beginnt mit der Entfernung des Wachstumsmediums und Zugabe von 100 µl /Well Beladungspuffer (5 µM BCECF-AM [2',7'-Bis-(Carboxyethyl)-5-(und -6)-Carboxyfluorescein, Acetoxymethylester] in 20 mM NH₄Cl, 115 mM Cholinchlorid, 1 mM MgCl₂, 1 mM CaCl₂, 5 mM KCI, 20 mM HEPES, 5 mM Glucose; pH 7,4 [mit KOH eingestellt]). Die Zellen werden dann 20 Minuten bei 37°C inkubiert. Diese Inkubation führt zur Beladung der Zellen mit dem fluoreszierenden Farbstoff, dessen Fluoreszenzintensität vom pHi abhängt, und mit NH₄Cl, was zu einer leichten Alkalinisierung der Zellen führt.
[Die nicht fluoreszierende Farbstoff-Vorstufe BCECF-AM ist als Ester membranpermeabel. Intrazellulär wird durch Esterasen der eigentliche Farbstoff BCECF freigesetzt, der nicht membranpermeabel ist.]

Nach dieser 20-minütigen Inkubation wird der Beladungspuffer, der NH₄Cl und freies BCECF-AM enthält, durch dreimaliges Waschen im Zellwasher (Tecan Columbus) mit jeweils 400 µl Waschpuffer (133,8 mM Cholinchlorid, 4,7 mM KCl, 1,25 mM MgCl₂, 1,25 mM CaCl₂, 0,97 mM K₂HPO₄, 0,23 mM KH₂PO₄, 5 mM HEPES, 5 mM Glucose; pH 7,4 [mit KOH eingestellt]) entfernt. Das in den Wells verbleibende Restvolumen beträgt 90 µl (50 - 125 µl möglich). Dieser Waschschritt entfernt das freie BCECF-AM und führt als Folge der Entfernung der externen NH₄⁺-lonen zu einer intrazellulären Ansäuerung (∼ pHi 6.3 - 6.4).
Da das Gleichgewicht von intrazellulärem NH₄⁺ mit NH₃ und H⁺ durch das Entfernen des extrazellulären NH₄⁺ und durch die nachfolgende, augenblicklich ablaufende Passage des NH₃ durch die Zellmembran gestört wird, führt der Waschprozess dazu, das intrazellulär H⁺ zurückbleibt, was die Ursache für die intrazelluläre Ansäuerung ist. Diese kann letztlich zum Zelltod führen, wenn sie lang genug anhält-An dieser Stelle ist es wichtig, dass der Waschpuffer natriumfrei (<1 mM) ist, da extrazelluläre Natrium-Ionen zu einer augenblicklichen Erholung des pHi durch die Aktivität der klonierten NHE-Isoformen führen würde.
Es ist ebenfalls wichtig, dass alle verwendeten Puffer (Beladungspuffer, Waschpuffer, Recoverypuffer) keine HCO₃-lonen enthalten, da die Anwesenheit von Bicarbonat zur Aktivierung störender bicarbonatabhängiger pHᵢ-Regulationssysteme führen würde, die in der parentalen LAP-1 Zelllinie enthalten sind.

Die Mikrotiterplatten mit den angesäuerten Zellen werden dann (bis zu 20 Minuten nach der Ansäuerung) zum FLIPR transferiert. Im FLIPR wird der intrazelluläre Fluoreszenzfarbstoff durch Licht mit einer Wellenlänge von 488 nm, das von einem Argon-Laser erzeugt wird, angeregt, und die Messparameter (Laserleistung, Belichtungszeit und Blende der im FLIPR eingebauten CCD-Kamera) werden so gewählt, dass das durchschnittliche Fluoreszenzsignal pro Well zwischen 30000 und 35000 relativen Fluoreszenzeinheiten liegt.

Die eigentliche Messung im FLIPR beginnt damit, dass Software-gesteuert alle zwei Sekunden eine Aufnahme mit der CCD-Kamera gemacht wird. Nach zehn Sekunden wird die Erholung des intrazellulärer pHs durch Zugabe von 90 µl Recoverypuffer (133,8 mM NaCl, 4,7 mM KCl, 1,25 mM MgCl₂, 1,25 mM CaCl₂, 0,97 mM K₂HPO₄, 0,23 mM KH₂PO₄, 10 mM HEPES, 5 mM Glucose; pH 7.4 [mit NaOH eingestellt]) mittels des im FLIPR eingebauten 96-Well-Pipettierers eingeleitet.
Als Positivkontrollen (100 % NHE-Aktivität) dienen Wells, denen reiner Recoverypuffer zugegeben wird, Negativkontrollen (0 % NHE-Aktivität) erhalten Waschpuffer. In allen anderen Wells wird Recoverypuffer mit der zweifach konzentrierten Testsubstanz hinzugegeben. Die Messung im FLIPR endet nach 60 Messpunkten (zwei Minuten).

Die Rohdaten werden in das Programm ActivityBase exportiert. Mit diesem Programm werden zunächst die NHE-Aktivitäten für jede getestete Substanzkonzentration und daraus die IC₅₀-Werte für die Substanzen berechnet. Da der Verlauf der pHᵢ-Erholung nicht während des ganzen Experiments linear ist, sondern am Ende aufgrund abnehmender NHE-Aktivität bei höheren pHi-Werten abfällt, ist es wichtig, für die Auswertung der Messung den Teil auszuwählen, in dem die Fluoreszenzzunahme der Positivkontrollen linear ist.

| Beispiel | NHE1-Hemmung IC₅₀ [nM] |
|---|---|
| 1 | 5.9 |

## Patentansprüche

1. 3,3-Difluor-cyclobutanol sowie dessen Salze.

2. 3,3-Difluoro-cyclobutylamin sowie dessen Salze.

3. Verwendung von 3,3-Difluor-cyclobutanol oder 3,3-Difluoro-cyclobutylamin sowie deren Salze als Syntheseintermediate.

4. Verwendung von 3,3-Difluor-cyclobutanol oder 3,3-Difluoro-cyclobutylamin sowie deren Salze zur Herstellung von N-[4-(3,3-Difluoro-cyclobutylamino)-5-methansulfonyl-2-methyl-benzoyl]-guanidin oder N-[4-(3,3-Difluoro-cyclobutylamino)-5-methansulfonyl-2-methyl-benzoyl]-guanidin sowie deren pharmakologisch verträgliche Salze.
